# EUROPÄISCHE PATENTSCHRIFT

(11) **EP 1 115 839 B1**
(45) Veröffentlichungstag und Bekanntmachung des Hinweises auf die Patenterteilung: **27.11.2002**
(21) Anmeldenummer: 99947330.9
(22) Anmeldetag: 13.09.1999
(51) Int. Cl.: C12M 1/22

(54) **Vorrichtung zur Aufnahme einer eine Zellkultur enthaltenden Flüssigkeit und Anordnung zum biologischen oder gentechnischen Behandeln von Zellen**
Cell culture device and apparatus for biological and genetical treatment of cells based thereon
Dispositif de culture céllulaire et appareil conténant ledit dispositif pour le traitement biologique et génetique des céllules

(30) Priorität: 24.09.1998 DE 29817223 U; 27.10.1998 EP 98120337
(43) Veröffentlichungstag der Anmeldung: 18.07.2001
(73) Patentinhaber: Marotzki, Stefan, Dr., 25436 Tornesch (DE)
(72) Erfinder: Marotzki, Stefan, Dr., 25436 Tornesch (DE)
(74) Vertreter: Glawe, Delfs, Moll & Partner
(86) Internationale Anmeldenummer: EP9906762
(87) Internationale Veröffentlichungsnummer: WO00017315

(56) Entgegenhaltungen:
- DE-A- 19 624 917
- DE-C- 479 304
- FR-A- 2 698 375
- US-A- 2 348 448
- US-A- 3 745 091
- US-A- 4 294 924
- US-A- 4 321 330
- US-A- 4 634 676

## Beschreibung

Für die molekularbiologische oder gentechnische Behandlung oder Untersuchung von Zellen verwendet man Gefäße (beispielsweise Petrischalen), die die Zellen und eine Reaktionsflüssigkeit aufnehmen und mit einem Deckel versehen werden können (Prospekt "Lab-Tek II" der Nalge Nunc International, Naperville; Prospekt "EasiSeal" der HYBAID Limited, Teddigton; Prospekt "Gene Frame" der Advanced Biotechnologies Ltd., Epsom; EP-A 611 598; US-A 4 634 676; US-A 3 745 091; DE-A 196 24 917; DE-C 479 304; US-A 2 348 448; US-A 4 294 924; US-A 4 321 330; FR-A 2 698 375). Wenn die Behandlung (beispielsweise bei der in situ Hybridisierung oder in situ PCR) den Austausch von Flüssigkeiten verlangt, kann dies bei den meisten bekannten Gefäßen nur manuell geschehen, weil es das Abnehmen und Aufsetzen eines Deckels voraussetzt. Wenn die Behandlung rasche Temperaturwechsel verlangt, muß dafür gesorgt werden, daß die Substratschicht im Gefäß sehr dünn ist, um dem Temperaturwechseln rasch und genau steuerbar folgen zu können. Diese Voraussetzungen können bei Behandlungen in automatischen Maschinen bislang erfüllt werden von solchen Gefäßen (Prospekt Lab-Tek II der Nalge Nunc International, Naperville; US-A 3 745 091), die aus einem Objektträger mit darauf lösbar angeordneten Wänden bestehen. In einer ersten Verfahrensstufe, die sich außerhalb der Automaten abspielt, werden die Gefäße mit den auf den Objektträgern aufgesetzten Wänden benutzt, wobei kein Flüssigkeitsaustausch stattfindet oder manuell durchgeführt werden muß. Sobald die automatische Behandlung beginnen soll, werden die Wände abgenommen und die Objektträger mit den daran haftenden Zellen in den Automaten eingebracht, in welchem der Flüssigkeitsaustausch dadurch stattfindet, daß die Objektträger gruppenweise in gegebenenfalls wechselnde Bäder eingesenkt werden. Falls die Zellen nicht oder nicht hinreichend am Objektträger adhärent sind, sondern ganz oder teilweise in Suspension sind, werden sie auf dem Objektträger zentrifugiert, damit sie nach dem Abnehmen des Mediums daran haften. Das ist nicht nur aufwendig, sondern hat auch den Nachteil, daß die Zellen deformiert werden und geringe morphologische Veränderungen unter dem Mikroskop nicht mehr identifiziert werden können. Außerdem sind die benötigten Zentrifugationsschritte nicht oder schwer automatisierbar.

Das Abnehmen der Wände von den Objektträgern kompliziert das Verfahren, insbesondere bei solchen Zellen, deren Haftung am Objektträger unzureichend ist. Schließlich kann die Aufhebung der Gefäßtrennung zwischen benachbarten Kulturen durch die Entfernung der Wände unerwünscht sein.

Der Erfindung liegt daher die Aufgabe zugrunde, eine Vorrichtung bzw. Anordnung gemäß dem Oberbegriff der Ansprüche 1 und 17 zu schaffen, die einen leichteren, insbesondere automatisierbaren Austausch der Behandlungsflüssigkeit erlauben. Dies gelingt dadurch, daß der Deckel des Gefäßes mindestens zwei Öffnungen für die Flüssigkeitszu- bzw. -Flüssigkeitsableitung enthält. Eine erfindungsgemäße Anordnung umfaßt eine Maschine mit Einrichtungen zum Austausch der Flüssigkeit, die für das Einbringen dieser Flüssigkeit in die eine bzw. für das Absaugen der Flüssigkeit aus der anderen Öffnung ausgebildet sind.

Die Erfindung gestattet den gegebenenfalls automatischen Flüssigkeitsaustausch ohne Beeinträchtigung der Zellkultur im vollständigen Schutz eines Gefäßes und gegebenenfalls unter Luftabschluß. Die Vorrichtung kann dank den mehreren Öffnungen auch als sogenannter Reaktor (Meenen et al.; "Semi Continuous Reactor System ...", Poster 1994 Biomaterials 21:905-908) verwendet werden.

Wenn verdrängte Flüssigkeit aufgefangen oder ein Vorrat von Flüssigkeit gebildet werden soll, der für den Austausch der im Behandlungsraum befindlichen Flüssigkeit vorgesehen ist, kann auf der Oberseite eine Wanne gebildet werden, die mit einer entsprechenden Deckelöffnung in Verbindung steht. Für die Flüssigkeit, die durch Diffusion oder Strömung in den Behandlungsraum gelangen soll, sollte der Wannenboden nicht niedriger als die Öffnungsmündung liegen. Dadurch, daß mehrere Öffnungen je Gefäß vorgesehen sind, kann ein Flüssigkeitsaustausch durch Strömung bewerkstelligt werden. Zu diesem Zweck sollten mehrere Öffnungen vorgesehen sein, von denen wenigstens eine mit einer von der Wanne bzw. den Wannen der übrigen Öffnungen getrennten Wanne verbunden ist, die nicht tiefer als die Öffnungsmündung liegt.

Die Wannen haben den Vorteil, daß die Einrichtungen der Maschine zum Einbringen von Flüssigkeit alternativ zum Einbringen in die Öffnungen oder in die jeweils zugeordneten Wannen ausgebildet sein können. Desgleichen können sie alternativ zum Absaugen von Flüssigkeit aus einer Wanne oder Öffnung ausgebildet sein.

Der Begriff Wanne soll in diesem Zusammenhang jede gefäßartige Vertiefung bezeichnen ohne eine Aussage über deren Breite oder Höhe, soweit dies nicht ausdrücklich angegeben ist.

Der Deckel kann - wie bekannt - zur Verdrängung von Luft und einem möglichen Überschuß an Reaktionsflüssigkeit durch die Öffnung ausgebildet sein. Der Deckel kann so weit innerhalb des Gefäßes abgesenkt werden, bis zwischen der Bodenplatte des Gefäßes und dem dieser gegenüberliegenden Deckelboden die gewünschte Dicke der Substratschicht erreicht ist, die von wenigen Hundertstel Millimetern bis zu einigen Millimetern reichen kann.

Insbesondere bei maschineller Behandlung der Vorrichtungen kann es zweckmäßig sein, mehrere Gefäße zusammenzufassen und mit einem gemeinsamen Deckel zu versehen, der jeweils die erforderlichen Öffnungen im Bereich jedes einzelnen Gefäßes aufweist.

Zur Vermeidung unerwünschten Totraums kann die Umfangsfläche des Deckels sich im wesentlichen formgleich und eng an die Innenfläche der Wände anschließen. Dieser Anschluß kann auch die Abdichtung des Deckels gegenüber den Wänden beinhalten, beispielsweise durch Glasschliff oder elastischen Druck einer anliegenden Kunststoffdichtung. Jedoch muß dies nicht der Fall sein, da zusätzlich zu einem engen, aber nicht dichten Anschluß der Umfangsfläche des Deckels an die Innenfläche der Wände eine besondere Dichtung vorgesehen sein kann, die beispielsweise mit dem Rand der Gefäßwände oder einem Absatz derselben zusammenwirken kann. In diesen Fällen ist es oft zweckmäßig, eine Halterung zur Aufrechterhaltung der Dichtposition des Deckels gegenüber dem Gefäß vorzusehen, beispielsweise einen Schraub-, Schnapp- oder Federverschluß; jedoch kann der dichte Anschluß des Deckels an die Gefäßwandung auch selbsthaltend sein, beispielsweise durch die Reibkräfte, die zwischen zwei Glasschliffflächen bestehen.

zweckmäßig ist es, wenn die Unterfläche des Deckelbodens etwa parallel zum Boden des Gefäßes verläuft, um eine im wesentlichen konstante Schichtdicke bewirken zu können. Der Deckel und das Gefäß können mit zusammenwirkenden Anschlagflächen versehen sein, die die Größe und Konstanz der Schichtdicke bestimmen.

Die Öffnungen des Deckels können mit einer Verschlußvorrichtung versehen sein. Jedoch besteht auch die Möglichkeit der Abdichtung mittels einer nachträglich aufgebrachten Ölschicht. Dies gilt auch für die Umfangsdichtung. In vielen Fällen ist dichter Abschluß lediglich gegenüber der äußeren Atmosphäre notwendig, um beispielsweise Verdunstung oder den Zutritt von Sauerstoff zu vermeiden. In diesen Fällen kann es genügen, wenn die Abdichtung nicht am Gefäß und Deckel, sondern von einer die Vorrichtung aufnehmenden Apparatur gebildet wird, beispielsweise einer Art Autoklaven, in welchem eine innere Atmosphäre herrscht, die nach den gewünschten Zwekken gewählt ist. Zur Vermeidung von Verdunstung kann diese beispielsweise hinreichende Feuchte aufweisen. Zur Vermeidung von Zutritt schädlicher Gase kann sie aus Stickstoff oder Edelgas bestehen.

Damit nicht nur die Luft im Bereich der Deckelöffnung entfernt wird, sondern auch derjenige Luftanteil, der zwischen der Umfangsfläche des Deckels und den Gefäßwänden enthalten sein mag, kann vorgesehen sein, daß die Deckelöffnung mit einer Steigstrecke versehen ist, deren Mündungshöhe mindestens etwa ebenso hoch ist wie das obere Ende des zwischen der Umfangsfläche des Deckels und den Gefäßwänden befindlichen Spalts. Bei langsamem Einsetzen des Deckels in das Gefäß sorgt dann der statische Druck der in der Steigstrecke der Deckelöffnung befindlichen Flüssigkeitssäule dafür, daß auch die Luft aus dem Umfangsspalt verdrängt wird.

Wenn die Zellen hinreichend fest dem Gefäßboden angelagert sind, kann die Überschußflüssigkeit ohne Gefahr von Zellverlust durch eine Deckelöffnung hindurch abgezogen bzw. verdrängt werden. Damit auch mit Zellen gearbeitet werden kann, die sich in Suspension befinden, kann die Deckelöffnung mit einem Sieb versehen werden, das die Zellen bei der Verdrängung der Flüssigkeit zurückhält.

Bei den aufgenommenen Zellen kann es sich um adhärente Zellen handeln, die am Boden der Zellkulturschale haften, oder um Suspensionszellen, die im Kulturmedium schwimmen. Alternativ können in der Schale auch Gewebeschnitte kultiviert werden. Allgemein können sowohl eukaryotische als auch prokaryotische Zellen in dem Gefäß kultiviert werden. Behandlungen und Untersuchungen, die mit der erfindungsgemäßen Vorrichtung durchzuführen sind, können beispielsweise alle Arten der in situ Hybridisierung und in situ PCR sein.

Die Deckelöffnungen werden zweckmäßigerweise innerhalb des Deckels in Abstand von dessen Rand vorgesehen. Jedoch soll nicht ausgeschlossen werden, daß eine Öffnung durch einen mindestens stellenweise vorgesehenen Abstand zwischen dem Deckelrand und den Gefäßwänden gebildet ist. Besonders vorteilhaft ist in diesem Zusammenhang eine Ausführung, bei der der Deckelrand einen von der unterseitigen Deckelplatte hochstehenden Kragen umfaßt, der in seiner Gesamtheit oder stellenweise den genannten Abstand zur Bildung einer Öffnung bildet und dessen oberer Rand mit den Gefäßwänden zur Bildung einer Dichtung zusammenwirkt. Während des Einsenkens des Dekkels können dann zwischen Deckel und Gefäß eingeschlossene Gas- und Flüssigkeitsanteile abströmen, und erst am Schluß dieses Vorgangs, wenn der Deckel seine Endposition erreicht, kommt die Dichtung zustande.

Wenn man mehrere Kulturen denselben thermischen Bedingungen unterwerfen will, ist es zweckmäßig, mehrere Gefäße miteinander zu verbinden. Dabei kann für jedes einzelne der Gefäße ein Deckel der oben bezeichneten Art vorgesehen sein. Jedoch ist es auch möglich, mehrere Deckel zu gemeinsamer Betätigung miteinander zu verbinden. Zwar ist es möglich, die Wände mehrerer Gefäße einstückig miteinander auszuführen, wobei entweder die Böden auch einstückig mit den Wänden verbunden sind oder von diesen, beispielsweise in der Form eines Objektträgers, trennbar sind.

Von besonderer Bedeutung ist im Zusammenhang der Erfindung die Möglichkeit, durch Wandbildung innerhalb eines und desselben Gefäßes mehrere Kammern schaffen zu können, die im Laufe der Behandlung einer Kultur je nach Wunsch zeitweise voneinander getrennt oder nicht getrennt sind. Zu diesem Zweck wird der Deckel mit einer Einrichtung zum Abteilen solcher Kammern voneinander versehen. Besonders vorteilhaft ist eine Ausführung, bei welcher ein engeres Gefäß so ausgebildet ist, daß es in einem weiteren Gefäß untergebracht werden kann. Der Deckel des weiteren Gefäßes ist dabei in der Regel so ausgebildet, daß er lediglich zum Verschluß des außerhalb des engeren Gefäßes befindlichen Bereichs des weiteren Gefäßes dient. Für den Verschluß des engeren Gefäßes ist dann gegebenenfalls ein besonderer Verschluß vorgesehen, für den auch das gilt, was weiter oben allgemein für den Verschluß der Gefäße angegeben wurde. Das heißt, außer einem einfachen luftdurchlässigen oder luftdichten Verschluß kann auch ein Deckel vorgesehen werden, der mit einer Öffnung zur Verdrängung der im engeren Gefäß anstehenden Atmosphäre und gegebenenfalls Überschußflüssigkeit eingerichtet ist und dessen Luftöffnung verschließbar ist. Es besteht aber auch die Möglichkeit, die Deckel des weiteren und des engeren Gefäßes konstruktiv zu gemeinsamer Betätigung miteinander zu verbinden.

Wenn in vorliegendem Zusammenhang von "einem" engeren Gefäß die Rede ist, so soll dies heißen, daß dies wenigstens eins ist; es können aber auch mehrere engere Gefäße in einem weiteren Gefäß vorgesehen sein. Die Anordnung eines engeren Gefäßes in einem weiteren Gefäß gibt die Möglichkeit, die Kulturen innerhalb des engeren und des weiteren Gefäßes genau denselben thermischen Bedingungen zu unterwerfen. Sie können gesonderte Böden haben. Von besonderer Bedeutung ist aber eine Ausführung, bei welcher das engere Gefäß den Boden mit dem weiteren Gefäß teilt. Dies gibt die Möglichkeit, eine und dieselbe, innerhalb des weiteren Gefäßes angelegte Kultur während aller oder eines Teils der Reaktionsschritte unterschiedlichen Reaktionsbedingungen (beispielsweise einer anderen Reaktionsflüssigkeit) zu unterwerfen. Dementsprechend wird die Wand des engeren Gefäßes lediglich in denjenigen Reaktionsschritten innerhalb der sonst einheitlichen Kultur eingesetzt, in denen die Reaktionsbedingungen unterschiedlich sein sollen, z. B. Positivkontrolle oder Negativkontrolle der PCR.

Es gibt Fälle, in denen das engere Gefäß während der gesamten Reaktion bzw. Reaktionsfolge in dem weiteren Gefäß verbleiben kann. In diesen Fällen kann es von vornherein fabrikmäßig mit dem Boden und dem weiteren Gefäß bleibend verbunden werden. In anderen Fällen wird die Kompartimentierung zu Beginn der Reaktion oder Reaktionskette gewünscht; dann kann das engere Gefäß zwar ebenfalls fabrikmäßig mit dem Boden des weiteren Gefäßes verbunden sein, ist aber lösbar, damit die Kompartimentierung im gewünschten Zeitpunkt aufgehoben werden kann. Die fabrikmäßige Anordnung hat den Vorteil, daß das engere Gefäß im Verhältnis zu den Wänden des weiteren genau positioniert werden kann und daher Gewähr dafür gegeben ist, daß der Deckel paßt. In wieder anderen Fällen will man Freiheit haben, die Kompartimentierung in einem beliebigen Zeitpunkt vornehmen zu können. In diesen Fällen ist die Wand des engeren Gefäßes vom Benutzer einsetzbar.

Wenn der Deckel das weitere Gefäß vollständig abschließen soll, muß er nicht nur mit seiner Umfangsfläche der Wand des weiteren Gefäßes angepaßt sein, sondern auch hinreichend dicht mit dem engeren Gefäß verbunden werden können. Dies kann dadurch geschehen, daß er einen der Form des engeren Gefäßes angepaßten Ausschnitt aufweist, der mit der gewünschten Dichtheit daran anschließen kann. Dieser Ausschnitt kann von einer Abströmöffnung gebildet sein. In einer anderen Ausführungsform ist der Deckel mit der Wandung des engeren Gefäßes einstückig verbunden, so daß die durch das engere Gefäß ermöglichte Kompartimentierung mit dem Einsetzen des Deckels zustande kommt. In beiden Fällen dient der Deckel als Halterung oder Führung beim Einsetzen und gegebenenfalls auch bei der Benutzung des engeren Gefäßes.

In manchen Fällen ist ein absolut dichter Abschluß der Wand des engeren Gefäßes gegenüber dem Boden des weiteren Gefäßes nicht erforderlich; das engere Gefäß kann dann einfach von einem Röhrchen gebildet sein, das vom Deckel gehalten wird und bis auf den Boden des weiteren Gefäßes hinabreicht. Wenn dichter Anschluß der engeren Gefäßwand an den Boden verlangt wird, kann der Unterrand der Wand des engeren Gefäßes mit einer Dichtung versehen sein, wobei die Wand zur Erzeugung der Dichtpressung durch den Deckel oder eine zusätzliche Klammer gegen den Boden gepreßt wird. Die Wand des engeren Gefäßes kann auch mit dem Boden verklebt werden. Im einfachsten Fall besteht die Gefäßwand des engeren Gefäßes aus einem Dichtungsring, der zwischen der Bodenplatte und dem Deckelboden eingelegt ist, an diese dicht anschließt und die von ihm eingeschlossene Kammer von dem umliegenden Raum abteilt. Damit der eingeschlossenen Kammer eine unterschiedliche Behandlung zuteil werden kann, muß sie durch eine in ihrem Bereich befindliche Deckelöffnung zugänglich sein, bei der es sich um die Abströmöffnung handeln kann.

Der Deckel kann nach der Erfindung auch mit weiteren Einrichtungen versehen sein, die für die Behandlung der Kulturen gewünscht sind. Für die Elektroporation können der Deckel und der Boden beispielsweise flächig ausgebildete Elektroden tragen und werden die zu behandelnden Zellen auf einer porösen Membran dazwischen eingesetzt.

Die Erfindung wird im folgenden näher unter Bezugnahme auf die Zeichnung erläutert, die vorteilhafte Ausführungsbeispiele schematisch veranschaulicht. Es zeigen:
- Fig. 1: eine Prinzipdarstellung,
- Fig. 2: eine Vorrichtung mit eingeschliffenem Deckel,
- Fig. 3: eine Vorrichtung mit elastisch dichtendem Deckel,
- Fig. 4: eine Vorrichtung mit Schraubdeckel,
- Fig. 5: eine Vorrichtung mit Rastdeckel,
- Fig. 6 - 8: unterschiedliche Ausführungsformen mit kompartimentierter Vorrichtung,
- Fig. 9: einen Teilschnitt, der eine Abdichtungsmöglichkeit veranschaulicht,
- Fig. 10: eine Vorrichtung mit Elektrode,
- Fig. 11: eine weitere kompartimentierte Vorrichtung,
- Fig. 12: eine Vorrichtung mit mehreren Gefäßen und gemeinsamem Deckel,
- Fig. 13: die Vorrichtung gemäß Fig. 12 mit besonderen Vertiefungen an den Deckelöffnungen,
- Fig. 14: die Vorrichtung gemäß Fig. 12 mit mehreren Öffnungen je Gefäß und
- Fig. 15 u. 16: ein Gefäß mit mehreren Öffnungen in unterschiedlichen Arbeitsstadien.

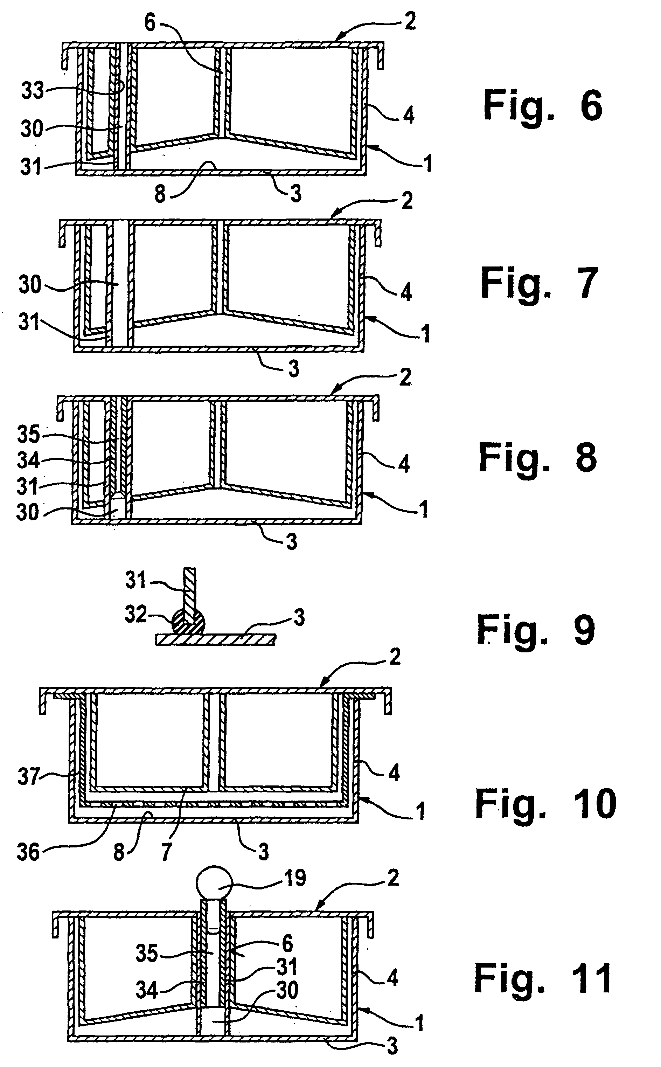

Die Vorrichtung besteht grundsätzlich aus dem Gefäß 1, beispielsweise einer Petrischale, und einem Deckel 2. Das Gefäß 1 hat eine Bodenplatte 3 und Wände 4. Diese können einstückig ausgeführt sein oder auch mehrteilig. In Fig. 3 ist angedeutet, daß die Bodenplatte von einem Objektträger 3' gebildet ist, an dem die Wände 4 in bekannter Weise mittels eines lösbaren Klebers 5 befestigt sind. Statt dessen kann auch ein Dichtungsring in Verbindung mit einer Einrichtung zum Halten der Wände unter Einschluß des Dichtungsrings am Objektträger verwendet werden. Gefäß und Deckel bestehen aus einem Werkstoff, der das Substrat und den ggf. durchzuführenden Prozeß nicht negativ beeinflußt, beispielsweise Glas oder geeignetem Kunststoff. Es kann sich um Einzelgefäße handeln; alternativ sind mehrere Gefäße als Gruppe einstückig zusammenhängend vorgesehen.

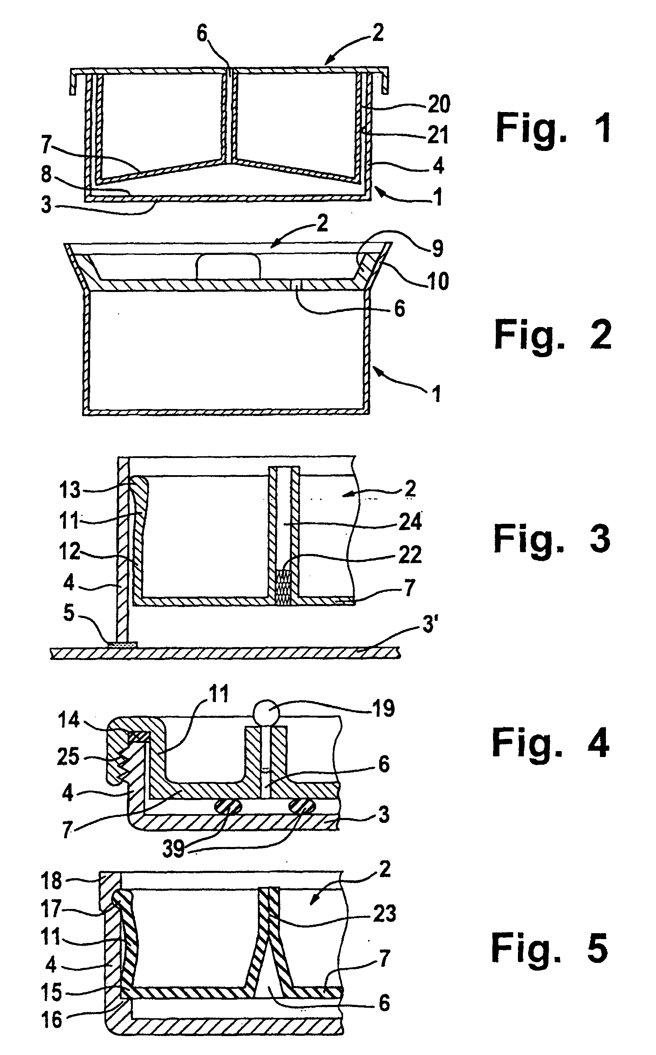

Will man die in der Schale befindlichen Zellen kultivieren oder molekularbiologisch oder gentechnisch behandeln oder untersuchen, verwendet man den Deckel 2, der die Schale dicht an den Wänden 4 abschließt. Er besitzt zwei Öffnungen, von denen in Fig. 1 bis 13 nur eine Öffnung 6 gezeigt ist. Durch sie kann die Atmosphäre und gegebenenfalls ein Teil der Flüssigkeit abströmen, die beim Einsetzen des Deckels in das Gefäß verdrängt wird. Die untere Mündung der Öffnung ist in dem in die Flüssigkeit eintauchenden Teil des Deckelbodens 7, und zwar vorzugsweise in dessen höchsten Bereich angeordnet.
Die Öffnung kann bei Bedarf nach Aufsetzen des Deckels verschlossen werden. Insoweit ist das Gefäß bekannt (FR-A 2698375). Der Abstand zwischen der Unterfläche des Deckelbodens 7 und der Oberseite 8 des Bodens beträgt zur Beobachtung in der Regel 20 µm, kann aber je nach Bedarf auch wesentlich größer oder kleiner sein.

Die jeweils gewählte Art der Abdichtung des Deckels 2 gegenüber den Wänden 4 hängt vom gewählten Material ab. Wird Glas verwendet, kann man sie mittels Schliff erreichen. Dies ist in Fig. 2 veranschaulicht. Der Deckel 2 und das Gefäß 1 sind mit übereinstimmend und komplementär geschliffenen Kragen 9, 10 versehen. Die Deckelöffnung 6 befindet sich an geeigneter Stelle innerhalb des vom Kragen umgebenen Deckelbereichs. Eine solche Form des Gefäßes und des Deckels kann auch bei anderen Werkstoffen als Glas verwendet werden.

Wird für die Gefäßwände und/oder den Deckel elastisch nachgiebiges Kunststoffmaterial verwendet, kann man die Dichtung durch federnde Anpressung einer Dichtkante erreichen, wie dies in Fig. 3 angedeutet ist. Die Wände 4 des z. B. aus Glas oder Kunststoff bestehenden Gefäßes verlaufen parallel zueinander und lotrecht zur Oberseite 8 des Bodens 3', beispielsweise zylindrisch. Der Deckel 2 besteht aus einem elastischen Kunststoff. Von seinem vorzugsweise ebenen Deckelboden 7 springt ein Kragen 11 auf, der im unteren Bereich 12 etwa parallel zu den Wänden 4 verläuft und mit diesem kein oder nur ein geringes Spiel einschließt. Der obere Rand 13 des Kragens 11 hat einen geringfügig größeren Durchmesser, so daß er beim Einsetzen in die Wand 4 elastisch zusammengedrückt wird und dadurch über seinen gesamten Umfang mit Vorspannung und dicht an der Innenfläche der Wand 4 anliegt. Die Stellung des Deckels im Gefäß wird durch seine Reibung an den Gefäßwänden gesichert.

Im Ausführungsbeispiel gemäß Fig. 4 schließt der Kragen 11 des Deckels ebenfalls mit nur geringer Spaltweite an die Wände 4 des Gefäßes an. Am freien Rand sind der Kragen 11 und die Wände 4 mit einem zusammenwirkenden Gewinde 25 versehen. Zwischen den Stirnflächen dieser Teile ist ein Dichtring 14 eingelegt. Auf den zwischen der Bodenplatte 3 und dem Deckelboden 7 eingelegten Ring 39 wird weiter unten eingegangen.

Statt des Schraubverschlusses kann auch ein irgendwie gearteter Schnappverschluß vorgesehen sein oder eine von außen auf den Deckel einwirkende, ihn in seiner Position haltende Klemme. Eine Ausführung mit Schnappverschluß ist in Fig. 5 dargestellt. Der Rand 15 des Deckelbodens 7 sitzt dichtend auf einem Absatz 16 der Wand 4 auf. In dieser Stellung wird er dadurch gehalten, daß der freie Rand 17 des Kragens 11 des Dekkels 2 rastend mit einer Umfangsnase 18 am freien Rand der Gefäßwände zusammenwirkt. Der Absatz 16 hat auch den Vorteil, daß er einen Anschlag bildet, der den gewünschten Abstand zwischen dem Deckelboden 7 und dem Gefäßboden 3 sichert.

Der Deckelboden 7 kann - wie in Fig. 1 gezeigt - so geformt sein, daß die Öffnung 6 an erhöhter Stelle des Bodens beginnt. Dadurch kann die restlose Beseitigung von Luftblasen erleichtert werden. Im allgemeinen ist dies aber nicht erforderlich. Restliche Lufteinschlüsse können mit einem Überschuß des Kulturmediums bzw. der Reaktionsflüssigkeit herausgespült werden.

In den Beispielen gemäß Fig. 1, 3 und 4 ist vorgesehen, daß die Öffnung 6 mit einem Steigrohr 24 verbunden ist, das mindestens ebenso hoch ist wie die zur Entlüftung des Spalts zwischen dem Deckelkragen 11 und den Gefäßwandungen 4 enthaltene Abströmstrecke. Durch den statischen Druck in dem Steigrohr kann gewünschtenfalls dafür gesorgt werden, daß Lufteinschlüsse aus dem Ringspalt zwischen der Umfangsfläche 20 des Deckels und der Innenfläche 21 der Gefäßwände vertrieben werden. Dieses Ziel kann man auch dadurch erreichen, daß man der Deckelöffnung einen so hohen Strömungswiderstand gegenüber Flüssigkeit verleiht und den Deckel so rasch einsetzt, daß der im Gefäß erzeugte Überdruck für die Verdrängung etwaiger Luft aus dem Ringspalt sorgt. Fig. 5 zeigt, daß sich die Abströmöffnung 6 nach oben hin zu einem Spalt 23 geringer Weite verengt, der als Drossel einen entsprechend hohen Strömungswiderstand hat. Dieser Spalt kann so ausgebildet sein, daß er sich in der Art eines Rückschlagventils beim Einsetzen des Deckels in das Gefäß unter elastischer Verformung des Materials für den Auslaß der Luft und von Überschußflüssigkeit öffnet, um sich anschließend unter der elastischen Rückstellkraft des Materials und/oder unter der Wirkung einer in der Zeichnung nicht dargestellten Klemme wieder luftdicht zu verschließen. Hingegen zeigt Fig. 4 für den Verschluß der Öffnung 6 einen Stopfen 19. Es besteht aber auch die Möglichkeit, die Öffnung mittels eines Öltröpfchens zu verschließen.

Wenn die Gefahr besteht, daß Zellen mit der verdrängten Flüssigkeit ausgeschwemmt werden, verschließt man die Deckelöffnung 6 mit einem Sieb 22, wie dies schematisch in Fig. 3 angedeutet ist.

Dank den mehreren vorgesehenen Deckelöffnungen 6 kann man die Vorrichtung als Bioreaktor verwenden, der von einem Reaktionsmedium durchströmt werden soll. In einem solchen Fall schließt man wenigstens eine Öffnung an einen Flüssigkeitszulauf und wenigstens eine andere Öffnung an einen Flüssigkeitsablauf an.

In manchen Fällen kann gänzlich auf einen luftdichten Abschluß zwischen dem Deckelrand und den Gefäßwänden verzichtet werden, nämlich dann, wenn innerhalb einer die Vorrichtung aufnehmenden Apparatur für eine unschädliche Atmosphäre gesorgt wird und diese gegenüber der äußeren Atmosphäre durch einen entsprechenden Abschluß der Apparatur geschützt wird, der in diesem Fall den an der Vorrichtung selbst vorgesehenen Abschluß vertritt.

Die Ausführungsbeispiele gemäß Fig. 6 bis 8 basieren auf dem Ausführungsbeispiel gemäß Fig. 1, könnten aber auch ohne weiteres von irgendeiner anderen Ausführungsform der oben beschriebenen Erfindung ausgehen. Sie veranschaulichen die Ausbildung einer engeren Kammer 30 innerhalb eines weiteren Gefäßes 1. Die die Kammer 30 bildenden Wände 31, bei denen es sich um ein einfaches Rohr, beispielsweise aus Glas oder Kunststoff, handeln kann, sitzen in jedem Fall mit ihren unteren Enden auf der Oberseite 8 der Bodenplatte 3 auf, um die Kammer 30 von ihrer Umgebung zu trennen. Wenn die Wandung 31 nicht einstückig mit der Bodenplatte 3 ausgebildet ist, kann sie mittels einer geeigneten Dichtung daran angeschlossen sein. Die Dichtung kann durch ein Klebmittel oder gemäß dem in Fig. 9 dargestellten Beispiel durch einen auf den unteren Rand fest und dicht aufgesetzten Elastomerstreifen 32 gebildet sein. In manchen Fällen genügt es, wenn sich die Unterkante der Wandung 31 ohne zusätzliches Dichtmittel auf die Oberseite 8 des Bodens 3 aufsetzt. In diesem Fall sowie bei Verwendung einer nicht verklebten Elastomerdichtung ist es zweckmäßig, die dichte Anlage an der Bodenoberseite 8 dadurch zu fördern, daß die Wand 31 gegen den Boden 3 vorgespannt ist. Wenn sie gemäß Fig. 7 mit dem Deckel 2 aus einem Stück besteht, kann diese Vorspannung dadurch unter Nutzung der elastischen Eigenschaften des Gefäßes oder Deckels erzeugt werden, daß die Länge der Wandung 31 ein gewisses Übermaß gegenüber dem Bodenabstand des Deckels aufweist und der Deckel fest auf das Gefäß 1 aufgesetzt ist, beispielsweise mit Hilfe eines Schraub-, Reib- oder Schnappverschlusses. Eine solche Vorspannung kann aber auch dann erzeugt werden, wenn - wie im Ausführungsbeispiel gemäß Fig. 6 - die Wandung 31 lösbar in einer Aufnahme oder Halterung 33 des Deckels 2 gehalten ist, indem von der Halterung 33 eine entsprechende Reibkraft auf die Wandung 31 ausgeübt wird. Die Halterung 33 schließt die Wandung 31 zweckmäßigerweise dicht ein. Sie wird beispielsweise von einer Bohrung gebildet, in der die röhrenförmige Wandung 31 eng passend geführt ist. Diese Bohrung kann, wie Fig. 11 zeigt, von einer Deckelöffnung 6 gebildet sein.

Wenn der Deckel 2 und die Wandung 31 gesonderte Teile sind, müssen sie im Gefäß 1 genau zueinander positioniert sein. Dies erreicht man am leichtesten dadurch, daß die Wandung 31 zunächst in den Deckel 2 eingesetzt wird und dieser dann zusammen mit der Wandung in das Gefäß 1 eingesetzt wird. Oder es wird erst der Deckel aufgesetzt und dann die Wandung 31 durch den Deckel eingesetzt. Wenn es erforderlich ist, die Kammer 30 innerhalb des Gefäßes 1 eznzurichten, bevor der Deckel 2 aufgesetzt wird, verwendet man am besten eine Positionierlehre für die Wandung 31, deren Abmessungen im Verhältnis zum Gefäß 1 denen des Deckels 2 gleichen. Diese Positionierlehre kann vor dem Einsetzen des Deckels 2 weggenommen werden, nachdem beispielsweise eine die wandeng 31 mit der Bodenoberseite 8 verbindende Klebeng erhärtet ist, oder die Positionierlehre bleibt an Ort und Stelle unterhalb des Dekkels 2, wenn dieser eingesetzt wird. Die Positionierlehre braucht im letzteren Fall kein gesondertes Teil zu sein; sie kann vielmehr konstruktiv mit der Wandung 31 vereinigt sein, indem diese beispielsweise eine Reihe von von ihr ringsum parallel zum Boden 3 des Gefäßes 1 oder zum Boden 7 des Dekkels vorspringenden Armen aufweist, die in positionierender Anlage an der Innenfläche 21 der Gefäßwände 4 enden.

Ebenso wie das Gefäß 1 mit einem mit einer Öffnung 6 ausgerüsteten Deckel 2 versehen ist, kann die Kammer 30 mit einem Verschlußorgan 34 versehen sein, das zur Begrenzung der Höhe des Substrats oberhalb der Oberfläche des Gefäßbodens 8 geeignet ist und einen Kanal 35 enthält, der wiederum mittels eines nicht gezeigten Stopfens verschließbar ist.

Ein weiteres Beispiel für Kompartimentierung ist in Fig. 4 veranschaulicht. Ein Dichtring 39 aus Elastomermaterial, beispielsweise ein handelsüblicher O-Ring, ist zwischen die Bodenplatte 3 und den Deckelboden 7 eingelegt. Das Ringmaterial ist so dick, daß es in der Endstellung unter elastischer Deformation dicht an der Bodenplatte 3 und dem Deckelboden 7 anliegt. Dadurch wird innerhalb des Dichtrings eine Kammer geschaffen, die von dem außerhalb des Dichtrings gelegenen Bereich abgetrennt ist. Durch die Abströmöffnung 6 des Dekkels oder irgendeine andere speziell zu diesem Zweck geschaffene Öffnung ist sie zugänglich, um eine andere Behandlung ihres Inhalts zu ermöglichen als im umliegenden Bereich. Der Ring 39 kann lose in das Gefäß 1 eingelegt werden, bevor der Deckel 2 aufgesetzt wird, wobei darauf geachtet werden muß, daß der von ihm umschlossene Bereich an der Stelle liegt, an der die Öffnung 6 zu erwarten ist. Damit die Kommunikation mit der Öffnung 6 gewährleistet ist und die Handhabung vereinfacht wird, kann der Ring auch mit dem Deckel 2 bleibend oder vorübergehend fest verbunden sein, beispielsweise durch eine Verklebung oder eine formschlüssige Halterung (in der Zeichnung nicht dargestellt).

Die erfindungsgemäße Kompartimentierung bietet die Möglichkeit, für einzelne oder mehrere Reaktionsschritte innerhalb der Kammer 30 etwas anderes zu tun als außerhalb derselben, wobei aber die thermischen Bedingungen dieselben bleiben. Auch können die Kulturen dieselben sein, wenn die Kammer 30 erst nach dem Anlegen der Kultur in dem Gefäß 1 eingesetzt wird. Beispielsweise kann in einem mehrschrittigen Verfahren in einem Schritt innerhalb der Kammer 30 doppelt soviel Enzym wie außerhalb der Kammer eingegeben werden. Danach wird die Kammerwand 31 wieder entfernt, und alle Zellen werden wieder identisch behandelt. Wenn mehrere derartige Einsetzkammern 30 in einem Gefäß verwendet werden, vermehren sich die Variationsmöglichkeiten entsprechend.

Sollen die Zellen in dem Gefäß einer Elektroporation oder Elektrofusion unterzogen werden, so werden im Deckel und Boden jeweils (in der Zeichnung nicht gezeigte) Metallelektroden vorgesehen. Die Zellen werden dann - wie in Fig. 10 gezeigt - auf einem porösen Boden 36 oder einer Membran eines Zellkultureinsatzes 37 zwischen Boden 3 und Deckel 2 eingebracht und einem elektrischen Feld ausgesetzt.

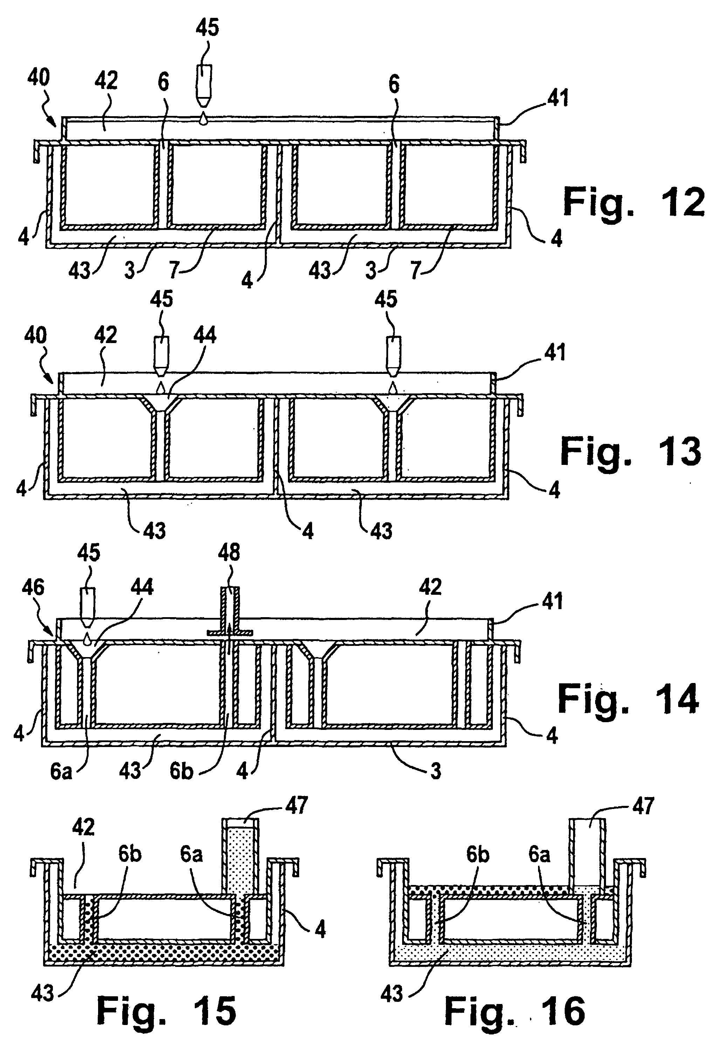

In den Ausführungen, die in Fig. 12 bis 14 gezeigt sind, ist vorgesehen, daß mehrere Gefäße mit Boden 3 und Wänden 4 einstückig zusammengefaßt sind. Es kann sich um mehr als die dargestellten zwei Gefäße handeln. Für mehrere dieser Gefäße ist ein gemeinsamer Deckel 40 vorgesehen, der auf irgendeine Weise, die oben erläutert wurde, mit den Wänden 4 der Gefäße abdichtend zusammenwirkt.

In der Ausführung gemäß Fig. 12 weist der Deckel 40 auf seiner Oberseite einen hochstehenden Rand 41 auf, der eine Wanne 42 umgrenzt, in welcher die Deckelöffnungen 6 münden. Zum einen fängt diese Wanne die Flüssigkeit auf, die beim Einsetzen des Deckels in das Gefäß abströmt. Zum anderen - dies ist der wichtigere Gesichtspunkt - kann die Wanne zur Zugabe von Flüssigkeit genutzt werden, die in die Behandlungsräume 43 überführt werden soll, um die Eigenschaften der dort befindlichen Flüssigkeit, in welcher sich die Zellen befinden, zu ändern. Es ist möglich, das Auffüllen bzw. Entleeren der Wanne durch einen geeigneten, bekannten Pipettierroboter zu bewerkstelligen.

Die Überführung der in der Wanne 42 befindlichen Flüssigkeit durch die Öffnungen 6 hindurch in die Kammern 43 kann durch Diffusion geschehen. Eine gemeinsame Wanne 42 für mehrere Öffnungen 6 von verschiedenen Behandlungsräumen 43 wählt man dann, wenn die Flüssigkeit in den verschiedenen Behandlungsräumen 43 in gleicher Weise geändert werden soll.

Wenn die Behandlungsflüssigkeit in benachbarten Behandlungsräumen 43 in unterschiedlicher Weise geändert werden soll, bedient man sich getrennter Wannen. Beispielsweise könnte in der Ausführungsform gemäß Fig. 12 auf der Oberseite des Dekkels in der Mitte zwischen den Mündungen der Öffnungen 6 noch eine weitere (nicht gezeigte) Wand 41 vorgesehen sein. Die Ausführung gemäß Fig. 13 erreicht das Ziel auf etwas andere Weise. Im Mündungsbereich jeder der Öffnungen 6 ist eine Vertiefung 44 im Boden der Wanne 42 vorgesehen. In die Vertiefungen 44 können unterschiedlichen Flüssigkeiten gegeben werden, die dann jeweils in die zugehörigen Behandlungsräume 43 hineindiffundieren. Die Vertiefungen 44 werden in den Ansprüche auch als Wanne bezeichnet.

Die Wannen geben die Möglichkeit, die Verfahrensschritte des Wechsels der Behandlungsflüssigkeit automatisch durchzuführen. Zu diesem Zweck wird eine geeignete Maschine (beispielsweise ein bekannter Pipettierroboter) mit schematisch bei 45 angedeuteten Düsen versehen, die einer programmierten Steuerung in der Weise unterliegen, daß sie zu einem gewünschten Zeitpunkt eine Flüssigkeit gewünschter Art und Menge in entsprechende Wannen geben.

Der Austausch der Behandlungsflüssigkeit kann durch erzwungene Strömung erfolgen. Zwei Ausführungsbeispiele von Vorrichtungen, die dafür geeignet sind, sind in Fig. 14 bis 16 dargestellt.

In Fig. 14 ist (wie in Fig. 12 und 13) angenommen, daß mehrere Gefäße mit Behandlungsräumen 43 einstückig zusammengeschlossen und mit einem einheitlichen Deckel 46 versehen sind. Jeder je einem Gefäß zugeordnete Teil des Deckels 46 weist zwei Öffnungen 6a, 6b auf, die auf der Oberseite des Deckels in einer Wanne 42 münden. Wenigstens die Öffnung 6a ist am oberen Ende mit einer Vertiefung 44 versehen. Zwar kann der Deckel gewünschtenfalls auch für den Austausch der Behandlungsflüssigkeit durch Diffusion verwendet werden; hauptsächlich ist er aber zum Zwangsaustausch der Behandlungsflüssigkeit geeignet. Zunächst wird in die Vertiefung 44 der Öffnung 6a mittels einer Düse 45 (die zu einem Pipettierroboter gehören kann) die neu einzubringende Behandlungsflüssigkeit eingegeben. Anschließend wird mittels eines Pipettierroboters oder einer auf die andere Öffnung 6b abgesenkten Saugdüse 48 die im Behandlungsraum 43 befindliche Behandlungsflüssigkeit abgesaugt, wodurch gleichzeitig die neu einzubringende Flüssigkeit blasenfrei in den Behandlungsraum nachgesaugt wird. Die Absaugmenge wird so genau dosiert, daß der Austausch gewährleistet ist. Die neu einzugebende Flüssigkeit kann im Überschuß vorhanden sein, wenn ein möglichst vollständiger Ersatz der zuvor im Behandlungsraum 43 befindlichen Flüssigkeit angestrebt wird und diese durch die nachfolgende Flüssigkeit ausgespült werden soll.

Das Ausführungsbeispiel gemäß Fig. 15 und 16 erlaubt einen Strömungsaustausch der Flüssigkeit auch ohne maschinelle Einrichtungen. Die Öffnung 6a, durch welche neue Flüssigkeit zugeführt werden soll, ist oberseits mit einer Wanne 47 geringen Querschnitts und verhältnismäßig großer Höhe verbunden, während die Öffnung 6b, durch die die zu ersetzende Flüssigkeit ausgestoßen wird, mit der Wanne 42 großen Querschnitts und verhältnismäßig geringer Höhe in Verbindung steht. Füllt man die hohe Wanne 47 - wie in Fig. 15 gezeigt - mit der neu einzubringenden Flüssigkeit, die gepunktet angedeutet ist, so bewirkt der statische Druckunterschied in den Wannen 47 und 42 das Austreiben der im Behandlungsraum 43 befindlichen, durch kleine Kreise symbolisierten Flüssigkeit. Gemäß Fig. 16 ist der Austausch dann vollendet, wenn in den Wannen 42 und 47 Niveauausgleich erreicht ist.

Es versteht sich, daß mehr Öffnungen als zwei vorgesehen werden können. Um einen möglichst vollkommenen Flüssigkeitsaustausch zu erreichen, kann es zweckmäßig sein, die Öffnung 6a für die neu einzubringende Flüssigkeit zentral vorzusehen und umfangsnah eine größere Anzahl von Austrittsöffnungen gleichmäßig zu verteilen.

Der Flüssigkeitsaustausch kann auch in umgekehrter Weise dadurch erfolgen, daß die neu einzugebende Flüssigkeit unter Überdruck in eine der Öffnungen eingegeben wird und dadurch die zu ersetzende Flüssigkeit aus der anderen Öffnung verdrängt wird. In diesem Fall kann es zweckmäßig sein, die Austrittsöffnungen, entsprechend dem Beispiel in Fig. 5, unter Überdruck öffnend und selbsttätig wieder schließend auszubilden.

## Patentansprüche

1. Vorrichtung zur Aufnahme einer eine Zellkultur enthaltenden Flüssigkeit mit einem eine Bodenplatte (3) und davon hochstehende Wände (4) aufweisenden Gefäß (1) und einem Deckel (46), der in das Gefäß (1) unter Verdrängung von Luft und gegebenenfalls Flüssigkeitsüberschuß einzusenken ist, **dadurch gekennzeichnet, daß** mindestens zwei Öffnungen (6a, 6b) im Deckel (46) vorgesehen sind, die zum Anschluß einer Flüssigkeitszu- und - Flüssigkeitsableitung ausgebildet sind.

2. Vorrichtung nach Anspruch 1, **dadurch gekennzeichnet, daß** eine oder mehrere Wände (31) zum Abtrennen mehrerer voneinander getrennter Kammern innerhalb des Gefäßes (1) vorgesehen sind.

3. Vorrichtung nach Anspruch 2, **dadurch gekennzeichnet, daß** ein engeres Gefäß (30) in dem weiteren Gefäß (1) angeordnet ist.

4. Vorrichtung nach Anspruch 3, **dadurch gekennzeichnet, daß** der Deckel (2) des weiteren Gefäßes (1) zum Verschluß lediglich des außerhalb des engeren Gefäßes (30) befindlichen Bereichs des weiteren Gefäßes (1) ausgebildet ist.

5. Vorrichtung nach Anspruch 3 oder 4, **dadurch gekennzeichnet, daß** der Deckel (2) einen dem Umfang des engeren Gefäßes (30) angepaßten Ausschnitt (33) aufweist.

6. Vorrichtung nach Anspruch 5, **dadurch gekennzeichnet, daß** der Ausschnitt (33) von der Deckelöffnung (6) gebildet ist.

7. Vorrichtung nach einem der Ansprüche 3 bis 6, **dadurch gekennzeichnet, daß** der Deckel (2) als Halterung (33) für das engere Gefäß (30) ausgebildet ist.

8. Vorrichtung nach Anspruch 7, **dadurch gekennzeichnet, daß** der Deckel (2) mit dem engeren Gefäß (30) zu gemeinsamer Handhabung verbunden ist.

9. Vorrichtung nach einem der Ansprüche 3 bis 7, **dadurch gekennzeichnet, daß** für das engere Gefäß (30) ein separater Deckel (34, 19) vorgesehen ist.

10. Vorrichtung nach einem der Ansprüche 2 bis 9, **dadurch gekennzeichnet, daß** zwischen Bodenplatte (3) und Deckelboden (7) ein eine gesonderte Kammer einschließender Ring (39) angeordnet ist und der Deckel im Bereich dieser Kammer eine Öffnung (6) aufweist.

11. Vorrichtung nach einem der Ansprüche 1 bis 10, **dadurch gekennzeichnet, daß** wenigstens ein Zellkultureinsatz (37) vorgesehen ist.

12. Vorrichtung nach einem der Ansprüche 1 bis 11, **dadurch gekennzeichnet, daß** mindestens eine Öffnung (6) mit einem Sieb (22) versehen ist.

13. Vorrichtung nach einem der Ansprüche 1 bis 12, **dadurch gekennzeichnet, daß** der Deckel (40, 46) auf seiner Oberseite eine mindestens die Mündung einer Öffnung (6) umgebende Wanne (42, 44, 47) aufweist.

14. Vorrichtung nach Anspruch 13, **dadurch gekennzeichnet, daß** der Wannenboden nicht niedriger als die zugehörige Öffnungsmündung liegt.

15. Vorrichtung nach Anspruch 13 oder 14, dadurch gekennkennzeichnet, daß wenigstens eine Öffnung (6b) mit einer von der Wanne (47) bzw. den Wannen der übrigen Öffnungen (6a) getrennten Wanne (42) verbunden ist, die nicht tiefer als die Mündung der Öffnung (6b) liegt.

16. Vorrichtung nach einem der Ansprüche 13 bis 15, **dadurch gekennzeichnet, daß** mehrere Gefäße einen gemeinsamen Deckel (40, 46) mit einer Wanne (42) aufweisen, die Öffnungen im Bereich von mehreren Gefäßen umfaßt.

17. Anordnung zum biologischen oder gentechnischen Behandeln von Zellen, bestehend aus wenigstens einem eine Bodenplatte (3) und davon hochstehende Wände (4, 31) aufweisenden Gefäß (1, 30) zur Aufnahme von Zellen und einer Behandlungsflüssigkeit sowie einer Maschine mit Einrichtungen zum Austausch der Flüssigkeit, **dadurch gekennzeichnet, daß** das Gefäß (1, 30) einen Deckel (2) aufweist, der wenigstens zwei Öffnungen (6a, 6b) enthält, und die Einrichtung (45, 48) zum Austausch der Flüssigkeit für das Einbringen in die eine bzw. Absaugen aus der anderen Öffnung (6a, 6b) ausgebildet sind.

18. Anordnung nach Anspruch 17, **dadurch gekennzeichnet, daß** der Deckel auf seiner Oberseite eine mindenstens die Mündung einer Öffnung (6a, 6b) umgebende Wanne (42, 44, 47) aufweist und die Einrichtungen (45, 48) zum Austausch der Flüssigkeit für das Einbringen in die eine bzw. Absaugen von der anderen Wanne (42, 44, 47) ausgebildet sind.

## Claims

1. Device for receiving a liquid containing a cell culture, with a vessel (1) having a base plate (3) and walls (4) rising upwards from the latter, and with a cover (46), which is to be lowered into the vessel (1) with displacement of air and, where appropriate, excess liquid, **characterized in that** at least two openings (6a, 6b) which are designed for the connection of a liquid admission line and a liquid discharge line are provided in the cover (46).

2. Device according to Claim 1, **characterized in that** one or more walls (31) are provided for separating off a plurality of separate chambers inside the vessel (1).

3. Device according to Claim 2, **characterized in that** a narrower vessel (30) is arranged in the wider vessel (1).

4. Device according to Claim 3, **characterized in that** the cover (2) of the wider vessel (1) is designed to close solely the area of the wider vessel (1) situated outside the narrower vessel (30).

5. Device according to Claim 3 or 4, **characterized in that** the cover (2) has a cutout (33) adapted to the circumference of the narrower vessel (30).

6. Device according to Claim 5, **characterized in that** the cutout (33) is formed by the cover opening (6).

7. Device according to one of Claims 3 to 6, **characterized in that** the cover (2) is designed as a holder (33) for the narrower vessel (30).

8. Device according to Claim 7, **characterized in that** the cover (2) is connected to the narrower vessel (30) for joint handling.

9. Device according one of Claims 3 to 7, **characterized in that** a separate cover (34, 19) is provided for the narrower vessel (30).

10. Device according to one of Claims 2 to 9, **characterized in that** a ring (39) enclosing a separate chamber is arranged between base plate (3) and cover base (7), and the cover has an opening (6) in the area of this chamber.

11. Device according to one of Claims 1 to 21, **characterized in that** at least one cell culture insert (37) is provided.

12. Device according to one of Claims 1 to 11, **characterized in that** at least one opening (6) is provided with a screen (22).

13. Device according to one of Claims 1 to 12, **characterized in that**, on its upper side, the cover (40, 46) has a trough (42, 44, 47) at least surrounding the mouth of an opening (6).

14. Device according to Claim 13, **characterized in that** the trough base is not lower than the associated mouth of the opening.

15. Device according to Claim 13 or 14, **characterized in that** at least one opening (6b) is connected to a trough (42) which is separate from the trough (47) or troughs of the other openings (6a) and which is not deeper than the mouth of the opening (6b).

16. Device according to one of Claims 13 to 15, **characterized in that** a plurality of vessels have a common cover (40, 46) with a trough (42) which has openings in the area of a plurality of vessels.

17. Arrangement for treating cells in biology or genetic engineering, consisting of at least one vessel (1, 30) which has a base plate (3) and walls (4, 31) rising upwards therefrom and is used for receiving cells and a treatment liquid, and a machine with means for exchanging the liquid, **characterized in that** the vessel (1, 30) has a cover (2) which has at least two openings (6a, 6b), and the means (45, 48) for exchanging the liquid are designed to introduce it into one and suction it out of the other opening (6a, 6b).

18. Arrangement according to Claim 17, **characterized in that** the cover has, on its upper side, a trough (42, 44, 47) at least surrounding the mouth of an opening (6a, 6b), and the means (45, 48) for exchanging the liquid are designed to introduce it into one and suction it out of the other trough (42, 44, 47).

## Revendications

1. Dispositif pour recevoir un liquide contenant une culture de cellules, avec un récipient (1) comportant une plaque de fond (3) et des parois (4) verticales sur celle-ci, ainsi qu'un couvercle (46) qui doit être enfoncé dans le récipient (1) en refoulant l'air et éventuellement l'excédent de liquide, **caractérisé en ce que** dans le couvercle (46) sont prévues au moins deux ouvertures (6a, 6b) qui sont conçues pour raccorder une arrivée et un départ de liquide.

2. Dispositif selon la revendication 1, **caractérisé en ce qu'**une ou plusieurs parois (31) sont prévues à l'intérieur du récipient (1), pour séparer plusieurs chambres séparées les unes des autres.

3. Dispositif selon la revendication 2, **caractérisé en ce qu'**un récipient étroit (30) est disposé dans le récipient plus large (1).

4. Dispositif selon la revendication 3, **caractérisé en ce que** le couvercle (3) du récipient plus large (1) est conçu pour fermer uniquement la zone de l'autre récipient (1) qui se situe à l'extérieur du récipient plus étroit (30).

5. Dispositif selon la revendication 3 ou 4, **caractérisé en ce que** le couvercle (2) présente une découpe (33) adaptée au périmètre du récipient plus étroit (30).

6. Dispositif selon la revendication 5, **caractérisé en ce que** la découpe (33) est formée par l'ouverture (6) du couvercle.

7. Dispositif selon l'une des revendications 3 à 6, **caractérisé en ce que** le couvercle (2) est réalisé en tant que fixation (33) pour le couvercle plus étroit (30).

8. Dispositif selon la revendication 7, **caractérisé en ce que** le couvercle (2) est relié au récipient plus étroit (30) pour leur manipulation commune.

9. Dispositif selon l'une des revendications 3 à 7, **caractérisé en ce qu'**un couvercle (34, 19) séparé est prévu pour le récipient plus étroit (30).

10. Dispositif selon l'une des revendications 2 à 9, **caractérisé en ce qu'**entre la plaque de fond (3) et le fond (7) du couvercle est disposé un anneau (39) enfermant une chambre séparée, et le couvercle présente une ouverture (6) dans la zone de cette chambre.

11. Dispositif selon l'une des revendications 1 à 10, **caractérisé en ce qu'**il est prévu au moins un insert (37) pour la culture de cellules.

12. Dispositif selon l'une des revendications 1 à 11, **caractérisé en ce qu'**au moins une ouverture (6) est pourvue d'un tamis (22).

13. Dispositif selon l'une des revendications 1 à 12, **caractérisé en ce que** sur sa face supérieure, le couvercle (40, 46) présente un bac (42, 44, 47) entourant au moins l'embouchure d'une ouverture (6).

14. Dispositif selon la revendication 13, **caractérisé en ce que** le fond du bac ne se situe pas plus bas que l'embouchure correspondante de l'ouverture.

15. Dispositif selon la revendication 13 ou 14, **caractérisé en ce qu'**au moins une ouverture (6b) est reliée à un bac (42) qui est séparé du bac (47) ou des bacs des autres ouvertures (6a) et qui ne se situe pas plus bas que l'embouchure de l'ouverture (6b).

16. Dispositif selon l'une des revendications 13 à 15, **caractérisé en ce que** plusieurs récipients comportent un couvercle (40, 46) commun avec un bac (42) qui entoure des ouvertures dans la zone de plusieurs récipients.

17. Dispositif pour le traitement biologique ou génétique de cellules, constitué d'au moins un récipient (1, 30), comportant une plaque de fond (3) et des parois (4, 31) verticales sur celui-ci, destiné à recevoir des cellules et un liquide de traitement, ainsi que d'une machine avec des dispositifs pour l'échange du liquide, **caractérisé en ce que** le récipient (1, 30) comprend un couvercle (2) qui contient au moins deux ouvertures (6a, 6b), et le dispositif (45, 48) pour l'échange du liquide est conçu pour l'introduction dans une ouverture (6a, 6b) ou l'aspiration depuis l'autre ouverture (6a, 6b).

18. Dispositif selon la revendication 17, **caractérisé en ce que** le couvercle comporte, sur sa face supérieure, un bac (42, 44, 47) entourant au moins l'embouchure d'une ouverture (6a, 6b) et les dispositifs (45, 48) pour l'échange du liquide sont conçus pour l'introduction dans un bac ou l'aspiration depuis l'autre bac (42, 44, 47).
